# EUROPEAN PATENT SPECIFICATION

(11) **EP 2 645 880 B1**
(45) Date of publication and mention of the grant of the patent: **12.04.2017**
(21) Application number: 11791511.6
(22) Date of filing: 28.11.2011
(51) Int. Cl.: A23L 27/00, A23L 27/24, A23L 27/26, A23L 13/00, A23L 13/30

(54) **PREPARATION OF SEASONING PRODUCTS**
HERSTELLUNG VON WÜRZPRODUKTEN
PRÉPARATION DE PRODUITS D'ASSAISONNEMENT

(30) Priority: 29.11.2010 CN 201010591214
(43) Date of publication of application: 09.10.2013
(73) Proprietor: Nestec S.A., 1800 Vevey (CH)
(72) Inventor: ULMER, Helge, Singapore 127159 (SG); LIAN HWEE PENG, Rebecca, Beijing 101300 (CN); QIN, Lan, Shanghai (CN); LI, Jingsen, Shanghai, PRC 200237 (CN)
(74) Representative: Mollet, Beat Max
(86) International application number: PCT/EP2011/071125
(87) International publication number: WO 2012/072549

(56) References cited:
- EP-A1- 0 417 481
- EP-A1- 1 104 654
- GB-A- 925 776
- US-A- 4 302 543

## Description

### TECHNICAL FIELD

The invention relates to a method for preparing seasoning products where solid state fermentation, hydrolysis and thermal reaction steps are carried out in a single apparatus.

### BACKGROUND

Seasoning products are powerful additives for improving food colour, odour, taste, and appetite stimulation. Such products are well-known and are extensively used in the food industry. Seasoning products may include sauces, such as soya sauce and Worchestershire (or Worcester) sauce, pastes such as miso paste, gravies, bouillons and other similar food flavouring agents. They may be products that are ready to use by adding directly to a prepared meal or to a food preparation being cooked. They may also be semi-finished seasoning products, which are generally considered to be processed products that are ready for further processing to give a seasoning having the desired flavour and aroma profile and intensity. Such semi-finished products include meat extracts, vegetable extracts, and other processed flavours.

Generally, a method for preparing a seasoning or a semi-finished seasoning product using microbiological fermentation technology comprises three steps, i.e. a fermentation step, a hydrolysis step and a thermal reaction step. For example, EP0417481, EP1104654 and US4302543 disclose such a method.

A solid state fermentation step is usually preferred over a liquid state (or submerged) fermentation step. This is because moulds can be grown in a solid state fermentation. In contrast, liquid state fermentations usually rely on the production of bacteria. Moulds, however, produce enzymes in a more concentrated manner. Bacteria are comparatively limited in the type and quantity of useful enzymes produced. Moreover, moulds are considered to be microbiologically safer than bacteria. Thus, enzyme production from moulds is usually more desirable than enzyme production from bacteria.

In conventional methods involving solid state fermentation, the three steps (fermentation, hydrolysis, thermal reaction) need to be carried out in different reaction containers or reactor units. Thus, the solid state fermentation step is carried out in a fermenter, the hydrolysis step is carried out in a hydrolysis tank or performed during fermentation, and the thermal reaction step is carried out in a thermal reaction container. As a consequence of carrying out these steps separately in different reaction containers, the reaction materials may need to be transferred several times, be reheated or cooled, and the intermediate products may need to be stored for some time. Additionally, the materials used and the reaction containers need to be sterilised separately which can result in unwanted thermal stresses. Conventional methods therefore have a number of drawbacks. They are time-consuming, important volatile flavour components (e.g. H₂S) may be lost, materials are more likely to be exposed to non-sterile conditions, and greater equipment investment and maintenance is required.

The applicant has now found a method of preparing semi-finished seasoning products that can be carried out in a single reaction vessel and therefore avoids all or some of the disadvantages of conventional methods.

It is therefore an object of the invention to provide a method for preparing a food seasoning product that at least goes part way to overcoming one or more of the above disadvantages, or at least to provide a useful alternative.

### SUMMARY OF THE INVENTION

The invention provides a method for preparing a food seasoning product comprising a solid state fermentation step, an hydrolysis step and a thermal reaction step, wherein the fermentation step, the hydrolysis step and the thermal reaction step are carried out in the same reaction vessel.

The fermentation step, hydrolysis step and thermal reaction step may be carried out in sequence or simultaneously.

The method preferably includes a milling step after the solid state fermentation step to reduce the size of particles before the hydrolysis step. The size of the particles is preferably reduced to less than about 1 mm in diameter.

It is preferred that the pressure in the reaction vessel is maintained in the range 0 to 1.2 bar (gauge pressure).

The reaction vessel and materials introduced into the reaction vessel for preparing the food seasoning product are preferably sterilised together.

In preferred embodiments of the invention, the temperature, humidity and stirring rate for preparing the food seasoning product are controlled using a computer system.

An apparatus for preparing a food seasoning product according to the method of the invention comprises:
a) a reaction vessel having a feed inlet capable of being opened and closed for introducing reaction materials into the reaction vessel, and a steam inlet capable of being opened and closed for introducing steam into the reaction vessel;
b) a milling unit for milling reaction materials after the fermentation step;
c) a stirrer located inside the reaction vessel for stirring reaction materials in the reaction vessel;
d) a sealing device for sealing the reaction vessel;
e) a gas exhaust outlet for exhausting gas from the reaction vessel and controlling pressure in the reaction vessel;
f) an outlet;
g) a temperature control device for controlling temperature in the reaction vessel; and
h) a temperature and/or humidity measuring device for measuring temperature and/or humidity in the reaction vessel.

Preferably the temperature control device is a jacket surrounding an outer wall surface of the reaction vessel, the jacket having a heating medium inlet capable of being opened and closed and a heating medium outlet capable of being opened and closed, wherein the jacket is used for controlling the temperature in the reaction vessel by circulating the heating medium through the jacket.

### BRIEF DESCRIPTION OF THE FIGURES

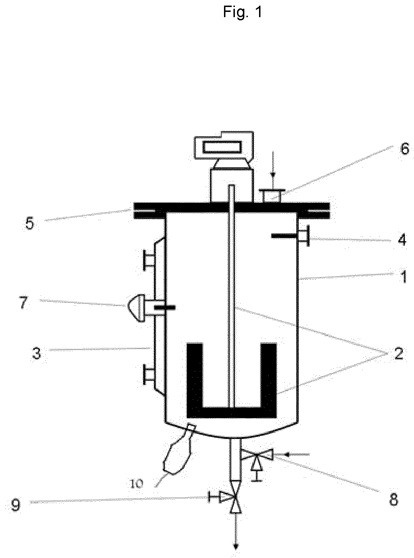

Figure 1 shows an apparatus for implementing the method of the invention.

### DETAILED DESCRIPTION

The invention relates to a method for preparing a food seasoning product comprising a solid state fermentation step, an hydrolysis step and a thermal reaction step, wherein the solid state fermentation step, the hydrolysis step and the thermal reaction step are carried out in the same reaction vessel of a reaction apparatus. The solid state fermentation step and the hydrolysis step may be conducted either in sequence or simultaneously.

The starting materials for preparing the seasoning product are introduced into the reaction vessel, and the materials are sterilised at the same time that the reaction container itself is sterilised.

Since the hydrolysis step cannot be efficiently carried out on large pieces of solid fermentation material, it is important to mill the solid after the fermentation step to particles of a size suitable for the hydrolysis step. The particles are preferably milled to around 1 mm or less in diameter. Thus, a key element of the invention enabling all three method steps to be carried out in a single reaction vessel is the incorporation of a milling unit so that the solid can be milled *in situ* within the reaction vessel. Without this milling unit, the solid material would have to be removed from the reaction vessel after the fermentation step, milled in a separate apparatus and then returned to the reaction vessel for hydrolysis and thermal reaction.

The pressure in the reaction vessel can be controlled. However, for safety reasons the pressure in the reaction vessel is preferably not more than 1.2 bar.

The temperature, humidity and stirring rate required for preparing the seasoning product may be automatically controlled by a computer system.

An important advantage of the invention is that, since the solid state fermentation step, the hydrolysis step and the thermal reaction step are all conducted in the same reaction vessel, there is no need for transferring any intermediate reaction products from one apparatus to another, nor any need for providing temporary storage of intermediate products. Thus, the method is energy efficient, time efficient, and the loss of volatile flavour components is minimised.

Moreover, the sterilisation efficiency of the method is improved because all the reaction materials can be introduced into the reaction vessel and the materials sterilised together with the reaction vessel in one sterilisation step. This is achieved by introducing steam at a sufficiently high temperature into the reaction vessel in the presence of the reaction materials.

In addition, because the solid state fermentation step, the hydrolysis step and the thermal reaction step are conducted in the same reaction container, the cost of equipment and maintenance is decreased.

A further advantage of the method of the invention is that solid state fermentation is a more efficient fermentation technique than submerged-type fermentation techniques (can yield high density intermediate products in the solid matrix). More diverse forms of microbiological strains and substrates are therefore able to be used in the method, compared to conventional food fermentation processes.

An apparatus for implementing the method of the invention comprises:
a) a reaction vessel having a feed inlet capable of being opened and closed for introducing reaction materials into the reaction vessel, and a steam inlet capable of being opened and closed for introducing steam into the reaction vessel;
b) a milling unit for milling reaction materials after the fermentation step;
c) a stirrer located inside the reaction vessel for stirring reaction materials in the reaction vessel;
d) a sealing device for sealing the reaction vessel;
e) a gas exhaust outlet for exhausting gas from the reaction vessel and controlling pressure in the reaction vessel;
f) an outlet;
g) a temperature control device for controlling temperature in the reaction vessel; and
h) a temperature and/or humidity measuring device for measuring temperature and/or humidity in the reaction vessel.

The temperature, humidity and stirring rate in the reaction vessel can be automatically controlled by a computer system, and can be adapted for the different preparation requirements of different seasoning products. The reaction vessel may also have a pressure gauge (for measuring pressure in the vessel).

The exhaust outlet may be any commonly used in the art, such as an exhaust outlet with a pressure reduction valve used in a pressure cooker.

The reaction vessel may also have an air inlet capable of being opened and closed, for introducing air into the reaction vessel.

Preferably, the temperature control device can be a jacket surrounding an outer wall surface of the reaction vessel. The jacket has a heating medium inlet capable of being opened and closed and a heating medium outlet capable of being opened and closed, wherein the jacket is used for controlling the temperature in the reaction vessel by circulating the heating medium through the jacket. The heating medium used in the jacket can be those commonly used in the art, and include water, steam, silicon oil, ethylene glycol, and the like.

The jacket preferably surrounds the outer wall surface of the reaction vessel in a spiral shape. The jacket can be made of various metal materials, but for optimal corrosion resistance and temperature control, the jacket is preferably made of stainless steel.

In preferred embodiments of the invention, the temperature, humidity, stirring rate and other parameters of the operation of the reaction vessel are computer controlled so that precise operating conditions can be selected for the particular seasoning product being prepared.

In one embodiment of the method, the fermentation step, the hydrolysis step and the thermal reaction step are continuously conducted in sequence in the same reaction vessel. Thus, in a first step, the materials for fermentation are fermented in the reaction vessel to obtain a fermentation product. The solid fermentation product is milled to a particle size suitable for the hydrolysis step, preferably where the particles have, on average, at least one dimension less than 1 mm, e.g. less than 1 mm in diameter. Water and/or a hydrolase are then introduced into the reaction vessel and the fermentation products are hydrolysed to give an hydrolysis product. Finally, the hydrolysis product undergoes a thermal reaction in the same reaction vessel to provide the seasoning product.

In another embodiment, the fermentation step and the hydrolysis step are conducted at the same time in the same reaction vessel, and then the hydrolysis products are subjected to the thermal reaction step.

In some embodiments of the invention, steam is introduced into the reaction vessel for sterilising the materials for fermentation and the reaction vessel prior to the fermentation step. Steam may also be introduced after the fermentation step in order to sterilise the fermentation products and the reaction vessel.

Examples of typical reaction materials that may be used in the method include protein sources such as wheat gluten, soybean, meat and meat extracts, vegetables and vegetable extracts, and carbohydrate sources such as sucrose, fructose, glucose and starch.

Examples of fermentation microorganisms that may be used include *Aspergillus oryzae, Saccharomyces sp., Lactobacillus sp.*, and *Bifidobacterium adolescentis*.

The invention is described in further detail with reference to Figure 1. The reaction vessel (1) is shown with a feed inlet (6) and an outlet (9). Both can be opened and closed to control the input and output of materials to and from the reaction vessel (1). The feed inlet (6) is located in a sealing device (5) at the top of the reaction vessel (1). Steam inlet (4) enables steam to be introduced. A stirrer (2) is used to mix the materials in the reaction vessel (1). The stirrer (2) comprises a shaft, with a stirring paddle connected to one end of the shaft, and is driven by an electric motor located on top of the reaction vessel (1). A milling unit (10) is arranged such that at least part of the milling unit (10) protrudes into, or is located inside, the reaction vessel (1) so that solid fermentation media can be milled to an appropriate particle size after the fermentation step and before the hydrolysis step. A temperature control device (3) is shown, as well as a temperature and humidity measuring device (7). The reaction vessel (1) is also equipped with an air inlet (8) for introducing air into the reaction vessel (1).

In preferred embodiments of the invention, opening or closing of the steam inlet (4), the feed inlet (6) and outlet (9), and the air inlet (8) and exhaust outlet are all automatically controlled using a computer system. Generally, temperature, humidity and stirring rate in the reaction vessel (1) are controlled within the ranges of 0-200 °C, 60-100 % RH (Relative Humidity), and 0-60 rpm, respectively.

In the examples below, various materials for preparing the seasoning product were introduced into reaction vessel (1) through feed inlet (6). Steam or air was introduced into reaction vessel (1) separately through steam inlet (4) or air inlet (8). Temperature, humidity and stirring rate in the reaction vessel (1) were automatically controlled by adjustment of the temperature control device (3), the steam inlet (4) and the stirrer (2) using a computer system. The temperature and humidity in the reaction vessel (1) were monitored in real-time via the temperature and humidity measuring device (7). In addition, the pressure in the reaction vessel (1) was maintained at about atmospheric pressure (about 1 bar at 20°C) by controlling the exhaust outlet using a pressure reduction valve.

The experimental results show that the seasonings produced by the method of the invention are similar in appearance to seasonings produced by conventional methods, but are stronger in meaty taste.

### EXAMPLES

The invention is further described with reference to the following examples. It will be appreciated that the invention as claimed is not intended to be limited in any way by these examples.

### Example 1: Preparation of concentrated soup base

Structured wheat gluten (6 kg) was introduced into a reaction tank and was mixed. Steam at 100 °C was then introduced into the reaction tank until the moisture content of the gluten reached 40% w/w. The mixture and the reaction tank together were pasteurised for 10 min using the steam maintained at a temperature of 100 °C. *Aspergillus oryzae* starter powder (0.03% w/w) was introduced into the reaction tank, and the mixture was fermented under aerobic and solid-state fermentation conditions at a temperature of 30 °C for 40 h while stirring at 5 rpm using conditioned air (100% RH, 30 °C). The solid mixture was milled to a particle size of about 0.2 to 0.9 mm in readiness for hydrolysis. Water (60% w/w) was then added into the reaction tank to hydrolyze the mixture at a temperature of 55 °C and a stirring rate of 45 rpm for 36 h. A thermal reaction was then initiated by adding xylose, salt and palm oil into the reaction tank. The reaction tank was maintained at 70 °C for 120 min while stirring at 50 rpm to ensure flavour formation. The mixture was exported from the reaction tank and maltodextrin added before drying using hot air to obtain a concentrated soup base in powder form. The concentrated soup base was found to have a similar appearance to concentrated soup bases produced by conventional methods (in which a fermentation step, hydrolysis step and thermal reaction were conducted in different reaction vessels), but the concentrated soup base of this example had a noticeably stronger meaty taste.

### Example 2: Preparation of liquid seasoning

Sugar (6 kg) and milled soybean (6 kg) were introduced into a reaction tank and were mixed. Steam at 100 °C was then introduced into the reaction tank until the moisture content of the mixture reached 60% w/w. The mixture and the reaction tank together were pasteurised for 10 min using the steam maintained at a temperature of 80 °C. *Saccharomyces sp.* (0.02% w/w) with proteolytic enzyme activity was added into the reaction tank, and the mixture was fermented under aerobic fermentation conditions at a temperature of 30 °C for 48 h while stirring at 10 rpm using conditioned air (100% RH, 30 °C). Steam at 80 °C was then introduced into the reaction tank, and the mixture and the reaction tank together were pasteurised for 30 min using the steam maintained at a temperature of 80 °C. The solid mixture was milled to a particle size of about 0.8 mm in readiness for hydrolysis. Phospholipase (0.3% w/w) and cellulase were added into the reaction tank to hydrolyze the mixture at a temperature of 50 °C and a stirring rate of 35 rpm for 36 h. A thermal reaction was initiated by adding glucose and salt into the reaction tank. The reaction tank was maintained at 90 °C for 60 min while stirring at 45 rpm to ensure flavour development. The mixture was exported from the reaction tank and starch added to obtain the liquid seasoning product. The liquid seasoning product was found to have a similar appearance to liquid seasoning products produced by conventional methods (in which a fermentation step, hydrolysis step and thermal reaction were conducted in different reaction containers), but the liquid seasoning product of this example exhibited a better taste.

### Example 3: Preparation of concentrated pork bouillon

Sugar (3 kg) and minced pork (4 kg) were introduced into a reaction tank and were mixed. Steam at 100 °C was then introduced into the reaction tank until the moisture content of the mixture reached 30% w/w. The mixture and the reaction tank together were pasteurised for 30 min using the steam maintained at a temperature of 127 °C. *Lactobacillus sp.* (0.04% w/w) with proteolytic activity was added into the reaction tank, and the mixture was fermented under anaerobic conditions at a temperature of 30 °C and a humidity of 90 % RH for 24 h while stirring at 5 rpm. The solid mixture was milled to a particle size of about 0.6 mm in readiness for hydrolysis. Water (60% w/w) was added into the reaction tank to hydrolyse the mixture at a temperature of 50 °C and a stirring rate of 40 rpm for 36 h. A thermal reaction was initiated by adding dextrin and salt into the reaction tank. The reaction tank was maintained at 105 °C for 90 min while stirring at 50 rpm to ensure flavour development. The mixture was exported from the reaction tank, starch added before vacuum drying to obtain the concentrated pork bouillon. The concentrated pork bouillon was found to have a similar appearance to concentrated pork bouillons produced by conventional methods (in which a fermentation step, hydrolysis step and thermal reaction were conducted in different reaction containers), but the concentrated pork bouillon of this example exhibited a stronger pork taste.

### Example 4: Preparation of concentrated seasoning granule

Fenugreek extract (3 kg), Ligusticum chuanxiong powder (0.6 kg), sucrose (2 kg) and water were introduced into a reaction tank and were mixed. Steam at 100 °C was then introduced into the reaction tank until the moisture content reached 70% w/w. The mixture and the reaction tank together were pasteurised for 30 min using the steam maintained at a temperature of 90 °C. Alcalase (commercial enzyme, 0.5% w/w), phospholipase (0.5% w/w), cellulase (0.5% w/w) and starter culture (*Bifidobacterium adolescentis*: 10⁸ cfu/ml, 1% w/w) were introduced into the reaction tank, and the mixture was fermented under anaerobic conditions and hydrolysed at the same time, at a temperature of 30°C, a humidity of 99 % RH and a stirring rate of 30 rpm for around 2 days. The mixture was then heated at 90 °C for 30 min while stirring at a speed of 45 rpm. The mixture was exported from the reaction tank, and salt (30% w/w) and MSG (40% w/w) added. The mixture was milled for 0.5 min at 5 °C to obtain a paste, and then granulated to a granule size of 2 mm, and dried using a fluidised bed dryer for 0.5 min with hot air at 105 °C. The final product of concentrated seasoning granule had a moisture content of 2.5 %. The concentrated seasoning granules were compared with products produced by conventional methods (in which fermentation step, hydrolysis step and thermal reaction are conducted in different reaction containers), and were found to be similar in appearance, but stronger in meaty taste.

## Claims

1. A method for preparing a food seasoning product comprising a solid state fermentation step, a hydrolysis step and a thermal reaction step, wherein the fermentation step, the hydrolysis step and the thermal reaction step are carried out in a same reaction vessel, wherein the reaction vessel has
a) a feed inlet capable of being opened and closed for introducing reaction materials into the reaction vessel, and a steam inlet capable of being opened and closed for introducing steam into the reaction vessel;
b) a milling unit for milling reaction materials after the fermentation steps;
c) a stirrer located inside the reaction vessel for stirring reaction materials in the reaction vessel;
d) a sealing device for sealing the reaction vessel;
e) a gas exhaust outlet for exhausting gas from the reaction vessel and controlling pressure in the reaction vessel;
f) an outlet;
g) a temperature control device for controlling temperature in the reaction vessel; and
h) a temperature and/or humidity measuring device for measuring temperature and/or humidity in the reaction vessel.

2. A method as claimed in claim 1, wherein the solid state fermentation step, hydrolysis step and thermal reaction step are carried out in sequence.

3. A method as claimed in claim 1, wherein the solid state fermentation step, hydrolysis step and thermal reaction step are carried out simultaneously.

4. A method as claimed in any one of claims 1 to 3, wherein the pressure in the reaction vessel is in the range 0 to 1.2 bar (gauge pressure).

5. A method as claimed in any one of claims 1 to 4, wherein the solid state fermentation step is carried out on a reaction mixture having a moisture content in the range 15 % to 75 % w/w, preferably 30 % to 70 % w/w.

6. A method as claimed in any one of claims 1 to 5, including a milling step after the solid state fermentation step to reduce the size of particles before the hydrolysis step.

7. A method as claimed in claim 6, wherein the size of the particles is reduced to less than 1 mm in diameter.

8. A method as claimed in any one of claims 1 to 7, wherein the reaction vessel and materials introduced into the reaction vessel for preparing the food seasoning product are sterilised together.

9. A method as claimed in any one of claims 1 to 8, wherein temperature, humidity and stirring rate for preparing the food seasoning product are controlled using a computer system.

10. Seasoning product obtainable by the method according to one of the claims 1 to 9.

## Patentansprüche

1. Verfahren zum Herstellen eines Nahrungsmittel-Würzproduktes, umfassend einen Feststoff-Fermentationsschritt, einen Hydrolyseschritt und einen thermischen Reaktionsschritt, wobei der Fermentationsschritt, der Hydrolyseschritt und der thermische Reaktionsschritt in einem gleichen Reaktionsgefäß ausgeführt werden, wobei das Reaktionsgefäß Folgendes aufweist
a) einen Zuführeinlass, der geöffnet und geschlossen werden kann, um Reaktionsmaterialien in das Reaktionsgefäß einzuleiten, und einen Dampfeinlass, der geöffnet und geschlossen werden kann, um Dampf in das Reaktionsgefäß einzuleiten;
b) eine Mahleinheit zum Zermahlen von Reaktionsmaterialien nach den Fermentationsschritten;
c) ein Rührwerk, das im Inneren des Reaktionsgefäßes angeordnet ist, um Reaktionsmaterialien in dem Reaktionsgefäß zu rühren;
d) eine Abdichtvorrichtung zum Abdichten des Reaktionsgefäßes;
e) einen Abgasauslass zum Ablassen von Gas aus dem Reaktionsgefäß und Steuern eines Drucks in dem Reaktionsgefäß;
f) einen Auslass;
g) eine Temperatursteuervorrichtung zum Steuern einer Temperatur in dem Reaktionsgefäß;
und
h) eine Temperatur- und/oder Feuchtigkeitsmessvorrichtung zum Messen einer Temperatur und/oder Feuchtigkeit in dem Reaktionsgefäß.

2. Verfahren nach Anspruch 1, wobei der Feststoff-Fermentationsschritt, der Hydrolyseschritt und der thermische Reaktionsschritt der Reihe nach ausgeführt werden.

3. Verfahren nach Anspruch 1, wobei der Feststoff-Fermentationsschritt, der Hydrolyseschritt und der thermische Reaktionsschritt gleichzeitig ausgeführt werden.

4. Verfahren nach einem der Ansprüche 1 bis 3, wobei der Druck in dem Reaktionsgefäß im Bereich von 0 bis 0,12 MPa (0 bis 1,2 Bar) (Manometerdruck) liegt.

5. Verfahren nach einem der Ansprüche 1 bis 4, wobei der Feststoff-Fermentationsschritt an einer Reaktionsmischung mit einem Feuchtigkeitsgehalt im Bereich von 15 % bis 75 % w/w, vorzugsweise 30 % bis 70 % w/w ausgeführt wird.

6. Verfahren nach einem der Ansprüche 1 bis 5, einen Mahlschritt nach dem Feststoff-Fermentationsschritt einschließend, um die Größe von Teilchen vor dem Hydrolyseschritt zu verringern.

7. Verfahren nach Anspruch 6, wobei die Größe der Teilchen auf weniger als 1 mm Durchmesser verringert wird.

8. Verfahren nach einem der Ansprüche 1 bis 7, wobei das Reaktionsgefäß und die Materialien, die zur Herstellung des Nahrungsmittel-Würzproduktes in das Reaktionsgefäß eingeleitet werden, zusammen sterilisiert werden.

9. Verfahren nach einem der Ansprüche 1 bis 8, wobei Temperatur, Feuchtigkeit und Rührrate zur Herstellung des Nahrungsmittel-Würzproduktes unter Verwendung eines Computersystems gesteuert werden.

10. Würzprodukt, das durch das Verfahren nach einem der Ansprüche 1 bis 9 erhältlich ist.

## Revendications

1. Procédé de préparation d'un produit d'assaisonnement alimentaire comprenant une étape de fermentation à l'état solide, une étape d'hydrolyse et une étape de réaction thermique, dans lequel l'étape de fermentation, l'étape d'hydrolyse et l'étape de réaction thermique sont réalisées dans le même récipient de réaction, dans lequel le récipient de réaction comporte
a) une entrée d'alimentation qui peut être ouverte et fermée pour introduire des produits de réaction dans le récipient de réaction, et une entrée de vapeur qui peut être ouverte et fermée pour introduire de la vapeur dans le récipient de réaction ;
b) une unité de broyage pour broyer les produits de réaction à l'issue des étapes de fermentation ;
c) un agitateur placé à l'intérieur d'un récipient de réaction pour agiter les produits de réaction dans le récipient de réaction ;
d) un dispositif de scellage pour sceller le récipient de réaction ;
e) une sortie d'évacuation de gaz pour évacuer le gaz du récipient de réaction et contrôler la pression dans le récipient de réaction ;
f) une sortie ;
g) un dispositif de contrôle de la température pour contrôler la température du récipient de réaction ;
et
h) un dispositif de mesure de la température et/ou de l'humidité pour mesurer la température et/ou l'humidité dans le récipient de réaction.

2. Procédé selon la revendication 1, dans lequel l'étape de fermentation à l'état solide, l'étape d'hydrolyse et l'étape de réaction thermique sont réalisées de façon séquentielle.

3. Procédé selon la revendication 1, dans lequel l'étape de fermentation à l'état solide, l'étape d'hydrolyse et l'étape de réaction thermique sont réalisées simultanément.

4. Procédé selon l'une quelconque des revendications 1 à 3, dans lequel la pression dans le récipient de réaction se situe dans la fourchette de 0 à 0,12 MPa (0 à 1,2 bar) (pression manométrique).

5. Procédé selon l'une quelconque des revendications 1 à 4, dans lequel l'étape de fermentation à l'état solide est réalisée sur un mélange réactionnel ayant une teneur en humidité dans la fourchette de 15 % à 75 % en poids, de préférence 30 % à 70 % en poids.

6. Procédé selon l'une quelconque des revendications 1 à 5, comprenant une étape de broyage à l'issue de l'étape de fermentation pour réduire la taille des particules avant l'étape d'hydrolyse.

7. Procédé selon la revendication 6, dans lequel la taille des particules est réduite à moins de 1 mm de diamètre.

8. Procédé selon l'une quelconque des revendications 1 à 7, dans lequel le récipient de réaction et les produits introduits dans le récipient de réaction pour la préparation du produit d'assaisonnement alimentaire sont stérilisés ensemble.

9. Procédé selon l'une quelconque des revendications 1 à 8, dans lequel la température, l'humidité et la vitesse d'agitation pour la préparation du produit d'assaisonnement alimentaire sont contrôlés par un système informatique.

10. Produit d'assaisonnement alimentaire qui peut être obtenu par le procédé selon l'une des revendications 1 à 9.
